# EUROPEAN PATENT APPLICATION

(11) **EP 2 368 543 A1**
(43) Date of publication of application: **28.09.2011**
(21) Application number: 10157859.9
(22) Date of filing: 25.03.2010
(51) Int. Cl.: A61K 9/16, A61K 9/20, A61K 31/397, A61K 31/405

(54) **Method of preparing a granulated pharmaceutical composition comprising simvastatin and/or ezetimibe**

(71) Applicant: KRKA, tovarna zdravil, d.d., Novo mesto, 8501 Novo mesto (SI)
(72) Inventor: Vrecer, Franc, 8351, Straza pri Novem mestu (SI); German, Tamara, 8000, Novo mesto (SI); Bukovec, Polona, 8000, Novo mesto (SI)
(74) Representative: UEXKÜLL & STOLBERG

(57) **Abstract**

A method of preparing a granulated pharmaceutical composition by wet granulation, which method comprises: (a) suspending one or more pharmaceutically active ingredients comprising statin or ezetimibe or both statin and ezetimibe in a granulating liquid to form a granulating suspension; (b) optionally subjecting the granulating suspension of step (a) to high pressure homogenisation; (c) adding the granulating suspension of step (a) or (b) to a composition comprising one or more pharmaceutically acceptable inactive ingredients; and (d) granulating the mixture of step (c), as well as pharmaceutical compositions obtained by this process.

## Description

The present invention relates to a novel method for preparing granulated pharmaceutical compositions comprising simvastatin and ezetimibe.

High blood or plasma cholesterol levels or hypercholesterolemia represent a common disease pattern preliminary in the well situated countries of the western hemisphere. Cholesterol may cause a "hardening of the arteries" so that arteries become narrowed and blood flow to the heart is slowed down or even blocked with the consequence that provision of oxygen to the organs is constrained. Hypercholesterolemia has been implicated in atherosclerosis, heart attack, and stroke and is one of several conditions that may lead to coronary artery disease, which is the leading cause of death in the United States, accounting for approximately 600,000 deaths per year. The risk group includes the overweight, smokers, those with a poor diet (e. g. one rich in saturated fats), those who take inadequate exercise and suffering from stress. For such risk individuals, as well as those tested and found to have unduly high plasma cholesterol levels, a variety of treatments have been proposed, e. g. changes in diet and habits, increased exercise, etc. However such treatments are not always easy to enforce and there exist also medicinal treatments which have been effective at reducing plasma cholesterol levels.

Commonly used compounds for the treatment or prevention of high cholesterol levels in individuals are the statins, such as atorvastatin, pravastatin, rosuvastatin, fluvastatin, simvastatin, and lovastatin. Among the group of statins, particularly atorvastatin and simvastatin exhibited good results in the treatment of conditions characterized by high cholesterol levels. Simvastatin has the following structure formula (I). Methods for its preparation are disclosed in e.g. in EP 0 033 538, EP 0 351 918, and EP 0 299 656.

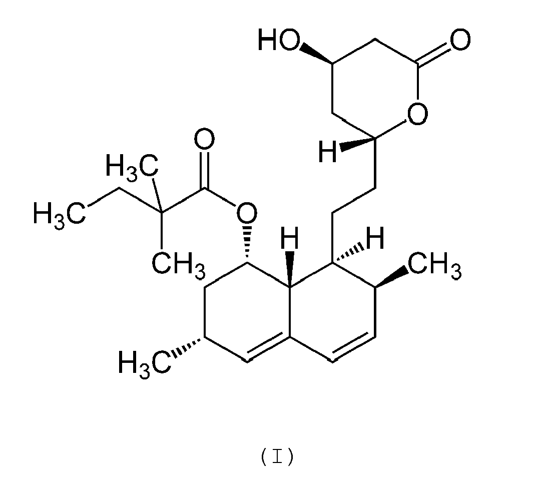

Simvastatin exerts a cholesterol reducing effect by inhibiting the conversion of 3-hydroxy-3-methylglutarylcoenzyme A (HMG-CoA) to mevalonate, an early step in the biosynthetic pathway of cholesterol. Additionally, simvastatin reduces the amount of very- low density lipoproteins (VLDL) and triglycerides (TG) and increases high-density lipoprotein cholesterol (HDL-C) and is thus capable to counteract diseases like atherosclerosis. Simvastatin was first marketed worldwide and sold under the trade name ZOCOR®

Also other compounds having a different mode of action with regard to a reduction of blood cholesterol levels have been proposed for use. Ezetimibe, which is disclosed in EP 0720 599 and identified by the structure formula (II): 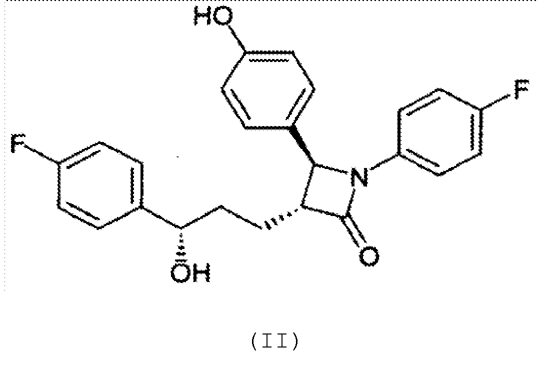 is such a compound. The mechanism of absorption and resorption inhibition of cholesterol of ezetimibe involves increased excretions of cholesterol and its intestinally generated metabolites with the faeces. This effect results in lowered body cholesterol levels, increased cholesterol synthesis, and decreased triglyceride synthesis. The increased cholesterol synthesis initially provides for the maintenance of cholesterol levels in the circulation, levels that eventually decline as the inhibition of cholesterol absorption and resorption continues. The overall effect of drug action is the lowering of cholesterol levels in the circulation and tissues of the body. It is market under the trade names ZETIA® and Ezetrol®. Polymorphic forms of this ezetimibe are for example described in WO 2005/009955, WO 2005/062897 and WO 2006/060808.

WO 2009/077573 discloses a process for the preparation of a suspension comprising non-micronized ezetimibe micro-particles having a small particle size and a high specific surface area wherein said ezetimibe micro-particles are prepared without micronization. The process prevents secondary agglomeration of ezetimibe micro-particles in the suspension.

Pharmaceutical compositions comprising ezetimibe and statins are disclosed for example in WO 2008/095263, WO 2009/024889, WO 2009/052246, WO 2009/147009 and WO 2010/021608.

In order to provide improved medication the art considered combination products, such as e.g. a combination of ezetimibe and simvastatin, which is marketed for example under the trade name VYTORIN® or Inegy®. For the time being, combinations comprising 10 mg of ezetimibe each and 10, 20, 40 and 80 mg simvastatin, respectively, are commercially available. Such a combination medicament has been proven effective in the treatment and/or prevention of atherosclerosis and related conditions.

Pharmaceutical compositions comprising ezetimibe and simvastatin are also disclosed in WO 2004/010993, W02006/134604; WO2007/003365, and US2009/0233898. All these references describe the preparation of the pharmaceutical formulations by wet granulation wherein a granulating solution is added to a dry mixture comprising ezetimibe, simvastatin and various excipients.

It is an object of the present invention to provide a new and simple method of preparing pharmaceutical formulations comprising ezetimibe and one or more statins as active ingredient(s) wherein the active ingredient(s) exhibit the required bioavailability. It is another object of the present invention to provide pharmaceutical compositions, in particular compositions wherein ezetimibe and statin are incorporated into a single dosage form, showing good solubility and a good dissolution rate of both active ingredients. Preferably the statin is simvastatin.

The object is solved by an innovative granulation process in which ezetimibe and/or simvastatin containing granulating suspension is subjected to a wet deagglomeration process using high pressure homogenization.

The present invention also relates to a method of preparing a granulated composition as described above and further processing the granulated composition to a tablet.

Moreover, the present invention concerns the granulated compositions and tablets obtained by the above methods.

A preferred method of preparing a granulated pharmaceutical composition by wet granulation comprises the following steps:
(a) suspending one or more pharmaceutically active ingredients comprising statin or ezetimibe or both statin and ezetimibe in a granulating liquid to form a granulating suspension;
(b) optionally subjecting the granulating suspension of step (a) to high pressure homogenization;
(c) adding the granulating suspension of step (a) or (b) to a composition comprising one or more pharmaceutically acceptable inactive ingredients; and
(d) granulating the mixture of step (c).
   The granulated mixture of step (d) can be further dried (step e). Preferably, the statin in the above process is simvastatin.

The granulated pharmaceutical compositions of the present invention comprise statin, ezetimibe or a combination thereof. Preferably, granulated compositions contain simvastatin and ezetimibe. Additional pharmaceutically active ingredients may be contained and introduced into the granulated compositions of the present invention at various stages; but in preferred embodiments statin and ezetimibe are the sole pharmaceutically active ingredients, more preferably simvastatin and ezetimibe are the active ingredients. The total amount of pharmaceutically active ingredients in the present pharmaceutical compositions ranges from 1 to 99 % by weight, particularly from 1 to 70 % by weight, more particularly from 5 to 40 % by weight, each based on the total weight of the pharmaceutical composition. The weight ratio of ezetimibe to simvastatin is typically within the range of from 1:1 to 1:8, more particularly 1:1 or 1:2 or 1:4 or 1:8 allowing to prepare final dosage forms comprising 10 mg of ezetimibe each and 10, 20, 40 and 80 mg simvastatin, respectively.

As used herein the terms "simvastatin" and "ezetimibe" include their pharmaceutically acceptable salts and their pharmaceutically acceptable derivatives.

In the suspending or dispersing step (a) one or more pharmaceutically active ingredients comprising simvastatin or ezetimibe or both simvastatin and ezetimibe are suspended (dispersed) in a granulating liquid to form a granulating suspension. The granulating suspension is then added to the composition comprising one or more pharmaceutically acceptable inactive ingredients (excipients) to initiate the wet granulation process.

Typically, the granulating liquid is a solvent which is preferably selected from water; one or more alcohols such as C₁ to C₄ alcohols, e.g. methanol, ethanol, and isopropyl alcohol; and mixtures of water and alcohol(s). The water is generally purified water or demineralized water. The granulating liquid may optionally contain one or more pharmaceutically acceptable inactive ingredients that have known functions of excipients used in the present compositions and described later. Preferably, the excipients contained in the granulating liquid are dissolved in the granulating liquid and more preferably the excipients are dissolved in the granulating liquid before the one or more pharmaceutically active ingredients are added and suspended therein. Examples of excipients that may be dissolved in the granulating liquid include binders. In preferred embodiments a binder is dissolved in the granulating liquid.

The pharmaceutically active ingredients that are suspended in the granulating liquid are preferably selected from simvastatin, ezetimibe and combinations of both simvastatin and ezetimibe. Typically the weight ratio between pharmaceutically active ingredients and binder in the granulating liquid calculated on dry weight of substances is in the range from 10:1 by weight to 0.1:1 by weight.

Additional pharmaceutically active ingredients can optionally be suspended in the granulating liquid or granulating suspension.

Optionally, the granulating suspension is subjected to high pressure homogenization in step (b) prior to the granulation, typically by passing it through a high pressure homogenizer. High pressure homogenization not only effects a homogenous dispersion of the pharmaceutically active ingredient(s) in the granulating liquid, but may also result in de-agglomeration or reduction of the particle size of the pharmaceutically active ingredient(s)

Simvastatin and ezetimibe are practically insoluble in water, which causes them to exhibit a low dissolution rate, which can further result in low bioavailability after oral ingestion. Generally, a reduction of particle size leads to better dissolution properties and therefore better bioavailability.

As for simvastatin it was found that with optionally passing the simvastatin granulating suspension through a high pressure homogenizer prior to the granulation, the particle size distribution of starting active ingredient simvastatin is not a limiting factor. Thus, both micronized and non-micronized simvastatin can be formulated in the present granulated pharmaceutical composition. "Micronized" as used herein is meant to describe a particulate material having a D90 of less than 25 microns more preferably less than 20 even more preferably less than 10 microns. D90 means that at least 90% by volume of the particles have a particle size below the specified value. "Non-micronized" as used herein is meant to describe a particulate material having a D90 of more than 40 microns. Preferably, the non-micronized simvastatin starting material used in the present method has a D90 of more than 50 microns. If non-micronized simvastatin is employed in the granulating suspension it is especially preferred to perform high pressure homogenization prior to granulation to reduce the particle size of the simvastatin.

Particle size can be determined by laser light scattering using e.g. a Malvern-Mastersizer Apparatus MS 2000 equipped with Hydro G dispersion cell unit. As the dilution medium deionised water is used and no sonication of the sample prior to the analysis is performed. Any laser diffraction method that would give an accurate result can be used if the correlation is performed.

In principle, micronized or non-micronized ezetimibe can be formulated in the present pharmaceutical composition. However, as for ezetimibe the inventors of the present invention surprisingly found out that besides small particle size and high specific surface area of ezetimibe particles the secondary agglomeration thereof during the process of the preparation of the pharmaceutical composition is a major drawback in providing required bioavailable product. Moreover, the inventors found that when reducing the particle size of ezetimibe by micronizing the chargeability of particles increases significantly what represents a lot of difficulties during the production of the pharmaceutical composition. Thus, it is preferred to use non-micronized ezetimibe as a starting material in the present method and it is even more preferred to perform high pressure homogenization prior to granulation to reduce the particle size of the non-micronized ezetimibe. In this way, secondary agglomeration of the ezetimibe particles in the suspension is prevented.

Surfactants such as sodium lauryl sulphate can be used to increase the bioavailability of low soluble active substance i.e. ezetimibe and also help prevent secondary agglomeration of ezetimibe particles in the suspension. However, it was surprisingly found, that there is no need for the addition of surfactants, and, therefore, in preferred embodiments of the present method no surfactant is added. Typically, the non-micronized ezetimibe starting material used in the present method has a D90 of from 30 to 70 microns and more preferably of from 40 to 50 microns.

According to the present invention micronized simvastatin in the final solid dosage form has a d90 of less than 20um.

After the optional high pressure homogenization the granulating suspension is added to a composition comprising one or more pharmaceutically acceptable inactive ingredients also referred to as "excipients" (step c). The terms "pharmaceutical acceptable ingredients" or "excipients" denote the additives used to convert pharmacologically active compounds into pharmaceutical dosage forms suitable for administration to patients. Suitable excipients can be solid, liquid or semisolid and convert during the process into the state that allows preparation of a solid pharmaceutical composition comprising ezetimibe and simvastatin. Examples of excipients for use in the present invention include binders, diluents, disintegrants, antioxidants, lubricants, glidants, sweeteners.

Typically, the granulating suspension is added to a composition comprising a mixture of various excipients. In preferred embodiments, the excipients mixture does not necessarily contain a binder because the binder has already been introduced into the granulating liquid. More preferably, the excipients mixture comprises excipients selected from diluents, disintegrants and antioxidants.

Mixing of the excipients can be performed in a conventional device used for mixing of powders, such as for example motionless (passive) mixers, fluidized bed, diffusion, biconicdiffusion, uniconic, biconic, turbular, cubic, planetary, Y-, V-shaped, and low shear or high shear mixers.

The excipients(s) are present in the granulated pharmaceutical composition according to the present invention in a typical amount of from 1 to 99 % by weight, particularly from 30 to 99 % by weight, more particularly from 60 to 95 % by weight, each based on the total weight of the pharmaceutical composition.

The particle size of the excipients used in the method according to the present invention is preferably within the range of D90 < 750 µm, preferably D90 < 500 µm. In general, smaller particle sizes improve homogeneity of the granulation.

The binder added to the granulating liquid and/or to the excipients mixture is preferably selected from the group consisting of polyvinylpyrrolidone (povidone), microcrystalline cellulose, hydroxyethylcellulose, hydroxypropylcellulose, hydroxypropylmethylcellulose and other cellulose ethers, starch, pregelatinised starch, and polymethacrylate, and mixtures thereof. It is advantageous to use a binder with a good water solubility, especially a very good solubility in cold water. Binders having very good solubility in water include for example povidone of different K-values and hydroxypropylcellulose, such as for example partially substituted poly(hydroxypropyl) ether of cellulose and/or low substituted poly(hydroxypropyl) ether of cellulose. Preferably, at least one binder is selected from polyvinylpyrrolidone and hydroxypropylcellulose.

Examples of suitable diluents include lactose, calcium carbonate, dibasic calcium phosphate anhydrous, dibasic calcium phosphate dehydrate (e.g. Emcompress®, available from JRS Pharma, Rosenberg, Germany), tribasic calcium phosphate, microcrystalline cellulose (e.g. Avicel® PH 101, Avicel® PH 102, both available from FMC Biopolymer, Philadelphia, USA), powdered cellulose, silicified microcrystalline cellulose (e.g. Prosolv®, available from JRS Pharma, Rosenberg, Germany), dextrates (for example Emdex®, available from JRS Pharma, Rosenberg, Germany), dextrose, fructose, glucose, lactitol, lactose anhydrous, lactose monohydrate, spray-dried lactose, magnesium oxide, magnesium carbonate, maltitol, maltodextrin, maltose, mannitol, starch, sucralose, sucrose, xylitol, and mixtures thereof. Also, special excipients for direct compression such as Cellactose® (a mixture of α-lactose monohydrate and cellulose powder, available from Meggle Pharma, Wasserburg, Germany) or StarLac® (a mixture of α-lactose monohydrate and maize starch, available from Meggle Pharma, Wasserburg, Germany) can be used. Preferably, lactose in any form and co-processed excipients containing lactose are not added in the present method. Lactose free compositions present a clear advantage since they can be administered to people with lactose intolerance.

Examples of suitable disintegrants include sodium carboxymethylcellulose, calcium carboxymethylcellulose, croscarmellose sodium (internally crosslinked sodium carboxymethylcellulose), crospovidone (crosslinked polyvinylpyrrolidone), starch and modified starches (such as sodium starch glycolate, e.g. Primojel®, available from DMV-Fonterra Excipients), low substituted hydroxypropylcellulose, magnesium aluminium silicate, calcium silicate, and mixtures thereof.

Examples of suitable antioxydants include natural antioxidants such as ascorbic acid, vitamin E, coffee acid, phenolic compounds such quercetin, rutin and/or synthetic antioxidants such as buthylhydroxyanizole (BHA), buthylhydroxytoluene (BHT.

Examples of suitable lubricants include magnesium stearate, sodium stearyl fumarate, sucrose esters of fatty acids, stearic acids, and mixtures thereof.

Examples of suitable glidants include silicon dioxide, talc, aluminium silicate, and mixtures thereof.

Examples of suitable sweeteners include aspartame, saccharin sodium, dipotassium glycirrhizinate, aspartame, thaumatin, and mixtures thereof.

Typically, the granulating suspension is added to the excipient(s) in step (c) by spraying it onto the excipients(s).

The one or more additional pharmaceutically active ingredients can be added in step (c).

The subsequent granulating step (d) is conducted in any appropriate equipment known in the art such as a high shear granulator and/or a fluid bed granulator.

Generally, the granulated mixture is preferably dried after granulation in step (e) in any pharmaceutical acceptable manner. Drying of the granulate for example can be performed in one of the following ways: trays, fluid bed, and microwave assist/vacuum/gas stripping (one pot processing).

The granulated pharmaceutical composition according to the present invention can be used to prepare a solid pharmaceutical dosage form selected from tablets, pills, powders, lozenges, sachets, soft and hard gelatine capsules, and suppositories. The dosage form is preferably suitable for oral application.

More preferably, the granulated pharmaceutical composition according to the present invention is further processed to form a tablet of desired shape such as oblong, round, capsule like with average weigh in the range 50 to 1000 mg. Rotary tablet presses known from the state of the art can be used for compression of granulate obtained according to present invention and optionally further excipients can be used in extragranular phase selected from diluents, disintegrants, lubricants, glidants admixed to the granulate. The one or more additional pharmaceutically active ingredients can be added in the compression step.

Optionally, compression is followed by coating. The tablet can be coated with conventional materials used for film coating, such as described in "Pharmaceutical Coating Technology", by G. Cole (ed.), 1995.

As already pointed out before, pharmaceutical compositions containing both simvastatin and ezetimibe are especially preferred. Three typical methods for preparing these granulated compositions are as follows:
In one embodiment of the present invention both simvastatin and ezetimibe are suspended in the granulating liquid and the composition is then further processed as described above, including the preferred embodiments.

In another embodiment ezetimibe is first suspended in the granulating liquid and the obtained suspension is then subjected to high pressure homogenization. After that, simvastatin is suspended in the granulating suspension and the composition is then further processed as described above, including the preferred embodiments.

In another embodiment simvastatin and ezetimibe are suspended in the granulating liquid and the obtained suspension is then subjected to high pressure homogenization.

In yet another embodiment simvastatin and ezetimibe are each separately processed to a first and second granulated composition as described above, including the preferred embodiments, and thereafter both granulates are mixed, optionally with the addition of further excipients(s). It is preferred to conduct the optional high pressure homogenization prior to granulation at least in the preparation of the granulated composition containing ezetimibe. Mixing of the two granulates and optional excipients(s) can be performed in a conventional device used for mixing of powders, such as for example those devices described above for mixing the excipients prior to granulation.

The pharmaceutical compositions containing both simvastatin and ezetimibe can be further processed to the desired solid dosage forms as described above, for example they can be compressed to tablets. In order to achive proper dissolution of active substances from tablets, disintegration time of tablets in water (determined according to Ph. Eur.) is preferably less than 5 minutes, more preferably less than 3 minutes. In order to have enough mechanical strength of tablets to be able to perform optional film coating of tablets, theirs friability determined according to Ph. Eur. should be less than 1%, preferably less than 0.5%.

Granulated composition of the present invention can be film coated. Thus, the process of the present invention can also comprise a coating step (f). The film coating is applied to enable visual differentiation among different strength by colour and/or to improve physical appereance, mechanical strength and/or stability of the composition. The film coating preferably comprises polymers soluble in water and/or gastric fluid selected from cellulose ethers having viscosity of 2 weight/vol.-% aqueous solution of less than 20 mPas such as hyprolose, hypromellose, derivatives of vinyl alcohol such as polyvinyl alcohol and/or graft copolymer of polyvinyl alciohol and polyethyleneglycol obtainable as Kollicoat IR and Kollicoat Protect from BASF Company (Ludwigshafen, Germany), salts and other derivatives of carboxymethyl cellulose such as sodium carboxymethyl cellulose. Further excipients selected from but not limited to plasticizers such as polyethylenglycol, triethylcitrate, dibutylsebacate, propylenglycol; antitacking agents such as talc, magnesium stearate, partial fatty acid esters of glycerol such as glycerol monostearate, insoluble pigments such as titanium and/or iron oxides, soluble colorants, soluble diluents such as lactose can be optionally incorporated into the film coating according to present invention. Film coating applied onto the core of solid composition obtained according to present invention can have decreased permeability for gases such as oxygen and/or moisture thus giving increased stability of coated solid composition. The film coating is applied onto the cores of solid composition of present invention by the processes and equipment known from the state of the art such as pan or fluid bed coating.

Coated or uncoated solid composition obtained according to the present invention can be packed into primary packaging which can preferably have decreased permeability for gases such as oxygen and/or moisture. Thus, according to one embodiment the process of the present invention also comprises a packaging step (g). Low gas such as moisture or oxygen permeable primary packaging materials such as aluminium or polychloro-3-fluoroethylene homopolymer/PVC laminate can be used with the thickness in the range 10 to 40 µm in case of Al/Al blisters and 10 to 110 µm in case of Al-polychloro-3-fluoroethylene homopolymer/PVC laminate blisters. Optionally, solid dosage forms of present invention can be packed into primary packaging with desiccant. Desiccant can be placed inside the packaging unit together with solid dosage forms such as tablets and/or in the closure system or can be incorporated into the walls of the primary packaging unit.

According to a preferred embodiment, contact between the pharmaceutical composition and environmental oxygen may be reduced or suppressed by either packaging the pharmaceutical composition under reduced pressure, packaging in an inert gas atmosphere, using a coating affording protection and stability of the pharmaceutical composition to environmental influences, or by using a packaging wherein the contact between the pharmaceutical composition and oxygen is reduced by the means of oxygen absorbers.

An atmosphere with reduced oxygen content or reduced oxygen partial pressure may be obtained by the use of an atmosphere of reduced pressure, e.g. by creating a partial vacuum by means of a suitable pump or by partial freezing or liquefying the atmosphere, by the use of an inert gas atmosphere, wherein as an inert gas nitrogen or argon is preferably used, or by the use of absorbents. Suitable absorbents may be selected from the group of commercially available absorbents such as humidity-activated oxygen absorbers, ultraviolet-radiation-activated absorbers, radiation-activated absorbers, microwaves-radiation-activated absorbers, absorbers activated by a combination of activation processes or absorbers without necessity of activation. Examples of commercially available absorbers are Ageless™ (Mitsubishi Gas Chemical), ATCO (Standa Industry), FreshPax™ (Multisorb Technologies), O-Buster™ (Hsiao Sung Non-Oxygen Chemical Co), Biotika Oxygen Absorber (Biotika) and the like.

The invention also provides a stabilised package of solid composition of present invention which is provided with a space for trapping and disposal of free oxygen. Moreover, if the active compounds of the present composition are exhibited to a reduced oxygen partial pressure, the formulation is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Al-polychloro-3-fluoroethylene homopolymer/PVC laminate blister.

Final packaging can optionally contain desiccant which can be used in blisters made of aluminium foil, incorporated into closure system of glass and/or polymeric containers or added directly into containers.

The pharmaceutical compositions prepared by the methods of the present invention are unique as they comprise the pharmaceutical active ingredient(s) with high bioavailability, good solubility and with a suitable dissolution rate.

The invention is now illustrated by the following examples which are not intended to limit the scope of the claims.

### Example 1

| | |
|---|---|
| Simvastatin | 40,00 mg |
| Ezetimibe | 10,00 mg |
| Povidone | 10,00 mg |
| Mannitol | 151,00 mg |
| Croscarmellose Sodium | 22,00 mg |
| Cellulose, microcrystalline | 163,00 mg |
| Sodium stearyl fumarate | 4,00 mg |

Ezetimibe was dispersed in the solution of povidone in purified water and passed through high pressure homogenizer. After the homogenization step simvastatin was dispersed and the obtained dispersion was sprayed onto the mixture of mannitol and part of croscarmellose sodium in the fluid bed granulator. After granulating and drying microcrystalline cellulose and part of croscarmellose sodium were added to obtain a compression mixture which was then compressed into tablets.

### Example 2

| | |
|---|---|
| Simvastatin | 40,00 mg |
| Ezetimibe | 10,00 mg |
| Povidone | 10,00 mg |
| Mannitol | 151,00 mg |
| Croscarmellose Sodium | 22,00 mg |
| Cellulose, Microcrystalline | 163,00 mg |
| Sodium stearyl fumarate | 4,00 mg |

Ezetimibe and simvastatin were dispersed in the solution of povidone in purified water and passed through high pressure homogenizer. The obtained dispersion was sprayed onto the mixture of mannitol and part of croscarmellose sodium in the fluid bed granulator. After granulating and drying microcrystalline cellulose and part of croscarmellose sodium were added to obtain a compression mixture. The obtained compression mixture was then compressed into tablets.

### Example 3

| | |
|---|---|
| Simvastatin | 40,00 mg |
| Ezetimibe | 10,00 mg |
| Lactose monohydrate | 144,00 mg |
| Povidone | 11,00 mg |
| Mannitol | 55,50 mg |
| Croscarmellose Sodium | 15,00 mg |
| Cellulose, Microcrystalline | 120,50 mg |
| Sodium stearyl fumarate | 4,00 mg |

Part of povidone was dissolved in purified water. Simvastatin was than dispersed in the obtained solution and passed through high pressure homogenizer. After the homogenization step the obtained dispersion was sprayed onto the mixture containing lactose monohydrate, part of microcrysralline cellulose and part of croscarmellose sodium and granulated in the fluid bed granulator. In a separate step ezetimibe was dispersed in the water solution of povidone and passed through high pressure homogenizer. Obtained dispersion was sprayed onto the mixture of mannitol and part of croscarmellose Sodium in the fluid bed granulator. After granulating and drying the two separate granulates obtained by above process were then mixed together with the addition of part of microcrystalline cellulose, part of croscarmellose sodium and sodium stearyl fumarate to obtain a compression mixture. Obtained mixture was then compressed into tablets.

### Example 4

| | |
|---|---|
| Simvastatin | 40,00 mg |
| Ezetimibe | 10,00 mg |
| Lactose monohydrate | 144,00 mg |
| Povidone | 11,00 mg |
| Mannitol | 55,50 mg |
| Croscarmellose Sodium | 15,00 mg |
| Cellulose, Microcrystalline | 120,50 mg |
| Sodium stearyl fumarate | 4,00 mg |

Part of povidone was dissolved in purified water. Simvastatin was then dispersed in the obtained solution and sprayed onto the mixture containing lactose monohydrate, part of microcrysralline cellulose and part of croscarmellose sodium and granulated in the fluid bed granulator. In a separate step ezetimibe was dispersed in the water solution of povidone and passed through high pressure homogenizer. Obtained dispersion was sprayed onto the mixture of mannitol and part of croscarmellose sodium in the fluid bed granulator. After granulating and drying the two separate granulates obtained by above process were then mixed together with the addition of part of microcrystalline cellulose, part of croscarmellose sodium and sodium stearyl fumarate to obtain a compression mixture. Obtained mixture was compressed into tablets.

### Example 5

| | |
|---|---|
| Simvastatin | 40,00 mg |
| Ezetimibe | 10,00 mg |
| Lactose monohydrate | 144,00 mg |
| Povidone | 11,00 mg |
| Mannitol | 55,50 mg |
| Croscarmellose Sodium | 15,00 mg |
| Cellulose, Microcrystalline | 120,50 mg |
| Sodium stearyl fumarate | 4,00 mg |

Simvastatin, lactose monohydrate, part of microcrystalline cellulose and part of croscarmellose sodium were mixed in high shear mixture. Part of povidone is dissolved in purified water and the obtained solution was sprayed onto the above mixture. The wet granules were dried in a fluid bed granulator. In a separate step Ezetimibe was dispersed in the solution of povidone in purified water and passed through high pressure homogenizer. The obtained dispersion was sprayed onto the mixture of mannitol and part of croscarmellose sodium in the fluid bed granulator and granulated. Both dried granulates were admixed with part of microcrystalline cellulose, part of croscarmellose sodium and sodium stearyl fumarate to form a compression mixture which was compressed into tablets.

### Example 6

| | |
|---|---|
| Atorvastatin calcium | 20,00 mg |
| Ezetimibe | 10,00 mg |
| Lactose monohydrate | 164,00 mg |
| Povidone | 11,00 mg |
| Mannitol | 55,50 mg |
| Croscarmellose Sodium | 15,00 mg |
| Cellulose, microcrystalline | 120,50 mg |
| Sodium stearyl fumarate | 4,00 mg |

Part of povidone was dissolved in purified water. Atorvastatin calcium was then dispersed in the obtained solution and sprayed onto the mixture containing lactose monohydrate, part of microcrysralline cellulose and part of croscarmellose sodium and granulated in the fluid bed granulator. In a separate step ezetimibe was dispersed in the water solution of povidone and passed through high pressure homogenizer. Obtained dispersion was sprayed onto the mixture of mannitol and part of croscarmellose sodium in the fluid bed granulator. After granulating and drying the two separate granulates obtained by above process were mixed together with the addition of part of microcrystalline cellulose, part of croscarmellose sodium and sodium stearyl fumarate to obtain a compression mixture. Obtained mixture was compressed into tablets.

### Example 7

| | |
|---|---|
| Atorvastatin calcium | 20,00 mg |
| Ezetimibe | 10,00 mg |
| Povidone | 10,00 mg |
| Mannitol | 171,00 mg |
| Croscarmellose Sodium | 22,00 mg |
| Cellulose, microcrystalline | 163,00 mg |
| Sodium stearyl fumarate | 4,00 mg |

Ezetimibe and atorvastatin calcium were dispersed in the solution of povidone in purified water and passed through high pressure homogenizer. The obtained dispersion was sprayed onto the mixture of mannitol and part of croscarmellose sodium in the fluid bed granulator. After granulating and drying microcrystalline cellulose and part of croscarmellose sodium were added to obtain a compression mixture. The obtained compression mixture was then compressed into tablets.

## Claims

1. A method of preparing a granulated pharmaceutical composition by wet granulation, which method comprises:
(a) suspending one or more pharmaceutically active ingredients comprising statin or ezetimibe or both statin and ezetimibe in a granulating liquid to form a granulating suspension;
(b) optionally subjecting the granulating suspension of step (a) to high pressure homogenisation;
(c) adding the granulating suspension of step (a) or (b) to a composition comprising one or more pharmaceutically acceptable inactive ingredients; and
(d) granulating the mixture of step (c).

2. The method of claim 1, wherein the granulating liquid is a solvent which is preferably selected from water, alcohol(s), and mixtures thereof.

3. The method of claim 1 or 2, wherein the granulating liquid contains a binder which is preferably dissolved in the granulating liquid.

4. The method of any of the preceding claims, wherein the granulating suspension is added to the composition comprising one or more pharmaceutically acceptable inactive ingredients by spraying it onto the composition.

5. The method of any of the preceding claims further comprising
(e) drying the granulated mixture of step (d).

6. The method of any of the preceding claims, wherein granulating is performed in a high shear granulator or fluid bed granulator.

7. The method of any of the preceding claims, comprising
(a) suspending statin and ezetimibe in the granulating liquid to form a granulating suspension;
(b) optionally subjecting the granulating suspension of step (a) to high pressure homogenization;
(c) adding the granulating suspension of step (a) or (b) to the composition comprising one or more pharmaceutically acceptable inactive ingredients; and
(d) granulating the mixture of step (c) to form a granulated pharmaceutical composition.

8. The method of any of claims 1 to 6, comprising
(a1) suspending ezetimibe in the granulating liquid to form a granulating suspension;
(b) subjecting the granulating suspension of step (a1) to high pressure homogenization;
(a2) suspending statin in the granulating suspension of step (b);
(c) adding the granulating suspension of step (a2) to the composition comprising one or more pharmaceutically acceptable inactive ingredients; and
(d) granulating the mixture of step (c) to form a granulated pharmaceutical composition.

9. The method of any of claims 1 to 6, comprising
(a1) suspending statin in the granulating liquid to form a granulating suspension;
(b) subjecting the granulating suspension of step (a1) to high pressure homogenization;
(a2) suspending ezetimibe in the granulating suspension of step (b);
(c) adding the granulating suspension of step (a2) to the composition comprising one or more pharmaceutically acceptable inactive ingredients; and
(d) granulating the mixture of step (c) to form a granulated pharmaceutical composition.

10. The method of any of claims 1 to 6 comprising
(I) preparing a first granulated composition comprising statin but not ezetimibe according to any of claims 1 to 6;
(II) preparing a second granulated composition comprising ezetimibe but not statin according to any of claims 1 to 6; and
(III) mixing the first and second granulated compositions, optionally with the addition of further pharmaceutically acceptable inactive ingredient(s).

11. The method of claim 10, wherein at least in the preparation of the second granulated composition comprising ezetimibe but not statin, the ezetimibe-containing granulating suspension of step (a) is subjected to high pressure homogenization.

12. The method of any one of the preceding claims wherein the statin is simvastatin.

13. A granulated pharmaceutical composition obtainable by the method of any of the preceding claims.

14. A method of preparing a tablet, which method comprises preparing a granulated pharmaceutical composition by the method of any of claims 1 to 12 and compressing the granulated pharmaceutical composition into a tablet.

15. The method of claim 14, wherein further excipients are admixed with the granulated pharmaceutical composition obtained by the methods of any claims 1 to 12 and wherein the total mixture is then compressed into solid dosage form.

16. A tablet obtainable by the method of claim 14 or 15.

17. Use of the granulated pharmaceutical composition of claim 13 for preparing a solid pharmaceutical dosage form selected from tablets, pills, powders, lozenges, sachets, soft and hard gelatine capsules, and suppositories.
